# EUROPEAN PATENT APPLICATION

(11) **EP 4 321 162 A1**
(43) Date of publication of application: **14.02.2024**
(21) Application number: 22382770.0
(22) Date of filing: 08.08.2022
(51) Int. Cl.: A61K 31/443, A61K 31/47, A61K 45/06, A61P 11/00, A61P 31/14

(54) **ANTIVIRAL AGENTS FOR THE TREATMENT OF INFECTIONS BY CORONAVIRUSES**

(71) Applicant: Consejo Superior de Investigaciones Científicas (CSIC), 28006 Madrid (ES)
(72) Inventor: ENJUANES SÁNCHEZ, Luis, Madrid (ES); HONRUBIA BELENGUER, José Manuel, Madrid (ES); SOLA GURPEGUI, María Isabel, Madrid (ES); ZÚÑIGA LUCAS, Sonia, Madrid (ES)
(74) Representative: Cueto, Sénida

(57) **Abstract**

The invention provides a CFTR modulator compound selected from the group consisting of 3-[6-[[1-(2,2-difluoro-1,3-benzodioxol -5-yl) cyclopropanecarbonyl] amino] -3-methylpyridin -2-yl]benzoic acid and/or N-(2,4-ditert-butyl-5-hydroxyphenyl)-4-oxo-1H-quinoline-3-carboxamide and pharmaceutically acceptable salts or deuterated derivatives thereof for use in the treatment of an infection produced by a Betacoronavirus in a subject and compositions comprising said compounds.

## Description

### Technical field

The invention provides compounds and compositions that are useful for treating a coronavirus infection in a subject.

### Background

Coronaviruses (CoVs) are cytoplasmic single-stranded, positive-sense RNA viruses and have the largest genomes known (around 30 kilobases (kb) among RNA viruses. CoVs affect animals and humans. Seven human CoVs (hCoVs) have been identified. Four of them (229E, OC43, HKU1 and NL63) are responsible of around 15-30% of common colds. In contrast, the other three zoonotic CoVs: the severe acute respiratory syndrome coronavirus (SARS-CoV), Middle East respiratory syndrome (MERS-CoV) and SARS-CoV-2 are deadly.

SARS-CoV was identified in the Guangdong province of China in 2002 and rapidly spread in more than 30 countries during six months. SARS-CoV infected more than 8,000 people with a mortality rate of around 10%. In 2012, MERS-CoV was identified in Saudi Arabia, 2,562 laboratory-confirmed cases and 890 deaths have been noticed as of May 2022 which means a fatality rate of 35. In the case of SARS-CoV-2, it was discovered in Decemberof 2019 and causes the disease known as COVID-19 producing the most important pandemic of the 21^{st} century. Since then, the European Center for Disease Control (ECDC) has reported 586 million cases worldwide and 6,4 million deaths with a mortality rate of 1.1 %. The study of the CoVs-host interaction is essential to develop antiviral therapies and safe vaccines in order to solve the international health emergency produced by the SARS-CoV-2 pandemic and also to prevent future pandemics produced by the emergence of other highly pathogenic CoVs.

CoVs have several proteins that include PDZ-binding motifs (PBMs) with the potential of influencing cell behavior to virus benefit. PBMs are specific sequences usually located in the last part of the protein and can interact with PDZ domains which are protein-protein interaction sequences consisting of around 80-90 amino acid residues. There are 266 PDZ domains which are part of more than 400 protein isoforms in the human genome. PBM core normally consists of the last four residues of the protein. There are different classes of PBMs depending on the nature of the PBM core sequence: Class I PBMs consist of the X-S/T-X-Φ_{-COOH} (where X could be any amino acid and Φ is a hydrophobic amino acid (normally V, I or L) sequence, class II PBMs have the X- Φ-X-Φ_{-COOH} residues and class III PBMs are formed by the X-D/E-X-Φ_{-COOH} sequence. Due to the functional versatility of proteins containing PDZ domains, viruses can modify a wide range of cellular functions through their PBMs to the viral replication and dissemination benefit, also affecting viral pathogenesis. Although all hCoVs E proteins have a PBM in their carboxy terminus, the PBM of SARS-CoV E protein is the only one that has been studied in detail. The role of the rest of E protein PBMs from different hCoVs in their virulence is still unknown. E protein PBMs sequence from other hCoVs could also be the reason of their attenuation or virulence as SARS-CoV E protein PBM is responsible for its virulence.

SARS-CoV E protein is a small integral membrane protein of 76 amino acids (a.a.) that includes a PBM which is formed by the last four a.a. of the carboxy terminus (-DLLV_ _{COOH}) which is the same PBM sequence as that of SARS-CoV-2. This, could be one of the reasons why SARS-CoV-2 is a deadly coronavirus. It has been described that SARS-CoV E protein PBM is a virulence factor. SARS-CoV E protein PBM induces an exacerbated pro-inflammatory response due to its binding to syntenin. The interaction of SARS-CoV E protein PBM and syntenin-1 leads to acute respiratory distress syndrome (ARDS), edema and death through the phosphorylation and activation of p38-MAPK, a protein involved in the expression of pro-inflammatory cytokines.

In the present invention, variants of SARS-CoV, MERS-CoV and SARS-CoV-2 that lack the E protein PBM have been generated by reverse genetics and their pathogenicity has been analyzed in mice. The PBM motifs of these three hCoVs have been shown to be virulence factors and participate in their replication. Furthermore, a collection of SARS-CoV mutants has been constructed in which the E protein PBM domain was replaced by the one derived from virulent or attenuated hCoVs, and their virulence was analyzed. A virulence gradient was observed, depending on whether the E protein PBM domain was derived from an attenuated or virulent hCoV. The gene expression patterns associated with the different PBM motifs in lungs of mice infected with SARS-CoV was analyzed by deep sequencing of the mRNAs expressed in lungs of infected mice, and it was observed that the E protein PBM motif of SARS-CoV and SARS-CoV-2 dysregulates the expression of genes related to ion transport and cell homeostasis. Specifically, a decrease of the mRNA expression of the cystic fibrosis transmembrane conductance regulator (CFTR), which is essential for edema resolution, was observed. The reduction of CFTR mRNA levels was associated with edema accumulation in lungs of mice infected with SARS-CoV or SARS-CoV-2. The effect of compounds that modulate the expression and activity of CFTR on the replication of SARS-CoV-2 was studied in cell cultures and it was observed that these compounds drastically reduced the production of SARS-CoV-2 and protects against its infection in mice model. These results showed the high relevance of the PBM motif in the replication and virulence of CoVs, and have allowed the identification of cellular targets for the selection of antivirals.

EP2225230B1 discloses a crystalline form of 3-(6-(1-(2,2-difluorobenzo[d][1,3]dioxol-5-yl)cyclopropanecarboxamido)-3-methylpyridin-2-yl)benzoic acid for use in the treatment of Cystic Fibrosis. However, this document does not disclose the use of this compound against viral infections.

EP2489659B1 discloses a N-(5-hydroxy-2,4-ditert-butyl-phenyl)-4-oxo-1H-quinoline-3-carboxamide for use in the treatment of Cystic Fibrosis. However, this document does not disclose the use of this compound against betacoronavirus mediated respiratory diseases.

### Description

In the present specification, the terms "treatment" and "treating" as used herein refer to the medical management of a subject with the intent to cure, ameliorate, stabilize, or prevent a disease, pathological condition, or disorder. These terms include active treatment, that is, treatment directed specifically toward the improvement of a disease, pathological condition, or disorder, and also includes causal treatment, that is, treatment directed toward removal of the cause of the associated disease, pathological condition, or disorder. In addition, this term includes palliative treatment, that is, treatment designed for the relief of symptoms rather than the curing of the disease, pathological condition, or disorder; preventative treatment, that is, treatment directed to minimizing or partially or completely inhibiting the development of the associated disease, pathological condition, or disorder; and supportive treatment, that is, treatment employed to supplement another specific therapy directed toward the improvement of the associated disease, pathological condition, or disorder. It is understood that treatment, while intended to cure, ameliorate, stabilize, or prevent a disease, pathological condition, or disorder, need not actually result in the cure, amelioration, stabilization or prevention. The effects of treatment can be measured or assessed as described herein and as known in the art as is suitable for the disease, pathological condition, or disorder involved. Such measurements and assessments can be made in qualitative and/or quantitative terms. Thus, for example, characteristics or features of a disease, pathological condition, or disorder and/or symptoms of a disease, pathological condition, or disorder can be reduced to any effect or to any amount. In the context of a subject suffering from Betacoronavirus infection, the terms "treatment" and "treating" refer to the medical management of a subject with the intent to cure, ameliorate, or stabilize Betacoronavirus infection.

As used herein, "subject" includes, but is not limited to, animals, plants, bacteria, viruses, parasites and any other organism or entity. The subject can be a vertebrate, more specifically a mammal (e.g., a human, horse, pig, rabbit, dog, sheep, goat, non-human primate, cow, cat, guinea pig or rodent), a fish, a bird or a reptile or an amphibian. The subject can be an invertebrate, more specifically an arthropod (e.g., insects and crustaceans). The term does not denote a particular age or sex. Thus, adult and newborn subjects, as well as fetuses, whether male or female, are intended to be covered. A patient refers to a subject afflicted with a disease or disorder. The term "patient" includes human and veterinary subjects. In one aspect, the compounds described herein can be administered to a subject comprising a human or an animal including, but not limited to, a mouse, dog, cat, horse, bovine or ovine and the like, that is in need of alleviation or amelioration from a recognized medical condition.

As used herein, "deuterated derivative(s)" means the same chemical structure, but with one or more hydrogen atoms replaced by a deuterium atom.

As used herein, "CFTR" means cystic fibrosis transmembrane conductance regulator.

As used herein, the term "modulator" refers to a compound that increases the activity of a biological compound such as a protein. For example, a CFTR modulator is a compound that increases the activity of CFTR. The increase in activity resulting from a CFTR modulator includes but is not limited to compounds that correct, potentiate, stabilize and/or amplify CFTR.

A first object of the invention relates to the use of CFTR modulators and pharmaceutically acceptable salts or a deuterated derivative thereof for use in the treatment of an infection produced by a Betacoronavirus in a subject.

In a particular embodiment, the CFTR modulators are selected from the group consisting of 3-[6-[[1-(2,2-difluoro-1,3-benzodioxol-5-yl)cyclopropanecarbonyl]amino]-3-methylpyridin-2-yl]benzoic acid (VX809) and/or N-(2,4-ditert-butyl-5-hydroxyphenyl)-4-oxo-1H-quinoline-3-carboxamide (VX770) and pharmaceutically acceptable salts or a deuterated derivative thereof.

Another object of the invention relates to the use of a CFTR modulators compound selected from the group consisting of VX809 and/or VX770 and pharmaceutically acceptable salts or a deuterated derivative thereof for use in the treatment of an infection produced by a Betacoronavirus in a subject.

VX809, also known as Lumacaftor stands for 3-[6-[[1-(2,2-difluoro-1,3-benzodioxol-5-yl)cyclopropanecarbonyl]amino]-3-methylpyridin-2-yl]benzoic acid and has the following structure:

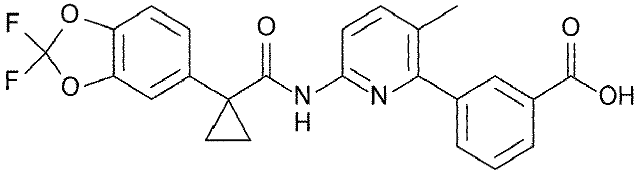

VX770, also known as Ivacaftor stands for N-(2,4-ditert-butyl-5-hydroxyphenyl)-4-oxo-1H-quinoline-3-carboxamide and has the following structure:

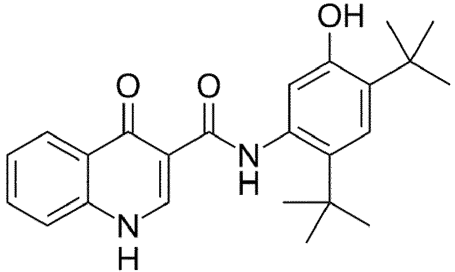

In a particular embodiment the term "treatment" means "preventing" which as used herein refers to administering a compound prior to the onset of clinical symptoms of a disease or conditions so as to prevent a physical manifestation of aberrations associated with the disease or condition. In the context of an infection caused by a betacoronavirus, the term "preventing" refers to administering a VX809 and/or VX770 prior to the onset of clinical symptoms of a betacoronavirus infection to prevent a physical manifestation of aberrations associated with a betacoronavirus infection.

In a preferred embodiment of the invention, a combination of VX809 and VX770 and pharmaceutically acceptable salts or deuterated derivative thereof is used for the treatment of an infection produced by a Betacoronavirus in a subject.

Whenever in this specification "deuterated derivative" is mentioned, it refers to both, deuterated derivative of any of the compounds or to deuterated derivative of an acceptable salt thereof.

As used herein, the term "a combination of" when referring to two or more compounds, agents mean the administration of two or more compounds to the subject having a Betacoronavirus infection prior to, concurrent with, or subsequent to each other.

The advantage of using a combination of both compounds is that there is a synergy in the reduction of the severity of the infection.

In a particular embodiment the concentration of VX809 administered to a subject is between 1000 mg/kg and 1 mg/kg of body weight, preferably between 500 mg/kg and 2 mg/kg, more preferably between 100 mg/kg and 3 mg/kg, even more preferably between 50 mg/kg and 4 mg/kg even more preferably between 25 mg/kg and 5 mg/kg, even more preferably between 20 mg/kg and 8 mg/kg and even more preferably between 15 mg/kg and 10 mg/kg.

In this specfication, when reference is made to a concentration given in mg/kg, it always means kg of body weight, unless otherwise specified.

In an additional particular embodiment, the concentration of VX770 administered to a subject is between 1000 mg/kg and 1 mg/kg, preferably between 500 mg/kg and 2 mg/kg, more preferably between 100 mg/kg and 3 mg/kg, even more preferably between 50 mg/kg and 4 mg/kg, even more preferably between 25 mg/kg and 5 mg/kg, and even more preferably between 10 mg/kg and 5 mg/kg.

In a preferred embodiment, the concentration of both VX809 and VX770 administered to a subject is between 25 mg/kg and 5 mg/kg for each compound. In another particular embodiment, the concentration of VX809 administered to a subject is about the double of the concentration of VX770 administered to said subject.

In an additional particular embodiment VX809 and VX770 are administered to a subject together at the same time or subsequent to each other.

In an additional particular embodiment, VX809 or VX770 is administered to a subject about 1, 2, 3, 4, 5, 6, 7, 8, 9, 10, 11 or 12 hour(s) after the other one is administered.

In an additional particular embodiment, the treatment with a CFTR modulator can happen before the subject shows the symptoms of a Betacoronavirus infection. For example, in a particular embodiment, a subject of high risk that have been exposed to a betacoronavirus infection and/or said subject has tested positive for a betacoronavirus infection. An expert in the field or a medical practitioner can determine which subjects are of high risk for instance those with advanced age, obesity, diabetes, asthma or chronic lung disease, sickle cell disease, or who are immunocompromised.

In a particular embodiment, the infection produced by a Betacoronavirus in a subject is a lung pathology or respiratory pathology.

Respiratory pathology and lung pathology are synonyms in the present specification.

For example, the risk of progression of the lung pathology or respiratory pathology to pneumonitis, pneumonia, acute respiratory distress syndrome, respiratory failure, lung microemboli, septic shock, organ failure, cytokine storm, and/or death can be inhibited by 10%, 20%, 30%, 40%, 50%, 60%, 70%, 80%, 90% or 100% relative to untreated control subjects of the same age and having the same comorbidities.

In an additional particular embodiment, the Betacoronavirus has a PBM domain formed by the last four amino acids of the carboxy terminus of the E protein selected from the group consisting of class I PBM, class II PBM or class III PBM. Class I PBMs consist of the X-S/T-X-Φ_{-COOH} sequence, class II PBMs have the X- Φ-X-Φ_{-COOH} residues and class III PBMs are formed by the X-D/E-X-Φ_{-COOH} sequence, wherein X could be any amino acid and Φ is a hydrophobic amino acid, preferably V, I or L.

In an additional particular embodiment, the PBM domain is a class II PBM, X- Φ-X-Φ_{-COOH} wherein X could be any amino acid and Φ is a hydrophobic amino acid, preferably V, I or L

In an additional particular embodiment, the PBM domain has the sequence -D-X₁-X₂-X₃-_{COOH}. Wherein X₁ is L or E; X₂ is L or W and X₃ is F or V, preferably X₁-X₂-X₃-_{COOH} is L-L-V-_{COOH} or X₁-X₂-X₃-_{COOH} is E-W-F-_{COOH} amino acid residues.

In an additional particular embodiment, the PBM domain has the sequence -DLLV-_{COOH} (SEQ ID NO: 1).

In an additional particular embodiment, the PBM domain has the sequence -DEWF-_{COOH} (SEQ ID NO: 2).

In a particular embodiment the Betacoronavirus is selected from the group consisting of *sarbecovirus* and *mervecovirus,* more preferably SARS-CoV, MERS-CoV and SARS-CoV-2, even more preferably SARS-CoV and SARS-CoV-2.

Another object of the invention relates to a pharmaceutical composition comprising one or more of the CFTR modulators described above plus:
- one or more additional active compound, and/or
- one or more pharmaceutically acceptable carrier, adjuvant, or vehicle.

By "pharmaceutically acceptable" is meant a material that is not biologically or otherwise undesirable, i.e., the material can be administered to a subject along with the selected compound without causing any undesirable biological effects or interacting in a deleterious manner with any of the other components of the pharmaceutical composition in which it is contained.

Also disclosed herein are pharmaceutical compositions comprising at least one of the compounds disclosed herein and/or at least one pharmaceutically acceptable salt thereof, which compositions may further include at least one additional active pharmaceutical ingredient and/or at least one carrier. Also disclosed are methods of treating the Betacoronavirus -mediated infection comprising administering at least one of the compounds disclosed herein and/or at least one pharmaceutically acceptable salt thereof, optionally as part of a pharmaceutical composition comprising at least one additional component, to a subject in need thereof.

As used herein - referring to pharmaceutical compositions - , the term "a combination of" when referring to two or more compounds, agents, and/or additional active pharmaceutical ingredients, means the administration of two or more compounds, agents, or active pharmaceutical ingredients to the subject prior to, concurrent with, or subsequent to each other.

Non-limiting examples of pharmaceutically acceptable salts derived from appropriate acids include: salts formed with inorganic acids, such as hydrochloric acid, hydrobromic acid, phosphoric acid, sulfuric acid, or perchloric acid; salts formed with organic acids, such as acetic acid, oxalic acid, maleic acid, tartaric acid, citric acid, succinic acid or malonic acid; and salts formed by using other methods used in the art, such as ion exchange. Non-limiting examples of pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxyethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, and valerate salts. Pharmaceutically acceptable salts derived from appropriate bases include alkali metal, alkaline earth metal, ammonium, and N+ (C1-4 alkyl)₄ salts. This disclosure also envisions the quaternization of any basic nitrogen-containing groups of the compounds disclosed herein. Suitable non-limiting examples of alkali and alkaline earth metal salts include salts of sodium, lithium, potassium, calcium, and magnesium. Further non-limiting examples of pharmaceutically acceptable salts include salts of ammonium, quaternary ammonium, and amine cations formed using counterions such as halide, hydroxide, carboxylate, sulfate, phosphate, nitrate, lower alkyl sulfonate and aryl sulfonate. Other suitable, non-limiting examples of pharmaceutically acceptable salts include besylate and glucosamine salts.

As described above, the pharmaceutically compositions of the present invention additionally can comprise one or more pharmaceutically acceptable carrier, adjuvant, or vehicle, which, as used herein, includes any and all solvents, diluents, or other liquid vehicle, dispersion or suspension aids, surface active agents, isotonic agents, thickening or emulsifying agents, preservatives, solid binders, lubricants and the like, as suited to the particular dosage form desired. Remington's Pharmaceutical Sciences, Sixteenth Edition, E. W. Martin (Mack Publishing Co., Easton, Pa., 1980) discloses various carriers used in formulating pharmaceutically acceptable compositions and known techniques for the preparation thereof. Except insofar as any conventional carrier medium is incompatible with the compounds of the invention, such as by producing any undesirable biological effect or otherwise interacting in a deleterious manner with any other component(s) of the pharmaceutically acceptable composition, its use is contemplated to be within the scope of this invention. Some examples of materials which can serve as pharmaceutically acceptable carriers include, but are not limited to, ion exchangers, alumina, aluminum stearate, lecithin, serum proteins, such as human serum albumin, buffer substances such as phosphates, glycine, sorbic acid, or potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, polyacrylates, waxes, polyethylene-polyoxypropylene-block polymers, wool fat, sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil; safflower oil; sesame oil; olive oil; corn oil and soybean oil; glycols; such a propylene glycol or polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of the formulator.

Each of CFTR modulator compounds, and pharmaceutically acceptable salts thereof described, and their deuterated derivatives herein independently can be administered once daily, twice daily, or three times daily. In some embodiments, at least one compound chosen from CFTR modulators compounds, and pharmaceutically acceptable salts thereof, and their deuterated derivatives is administered once daily. In some embodiments, at least one compound chosen from CFTR modulators compounds, and pharmaceutically acceptable salts thereof, and their deuterated derivatives are administered twice daily.

In a particular embodiment, the invention involves administering to a subject suffering from a Betacoronavirus infection an effective amount of a composition comprising an effective amount of a compound that modulates CFTR activity. The amount of compound that modulates CFTR activity can be an amount from a low amount of about 0.1 µg/ml of solution, about 0.2 µg/ml, or about 0.5 µg/ml, to a high of about 100 µg/ml, about 200 µg/ml, or about 500 µg/ml. For example, the amount of compound that modulates CFTR activity can be from about 0.1 µg/ml to about 100 µg/ml, from about 0.2 µg/ml to about 200 µg/ml, from about 0.5 µg/ml to about 500 µg/ml, from about 0.1 µg/ml to about 10 µg/ml, from about 0.1 µg/ml to about 20 µg/ml, from about 0.1 µg/ml to about 50 µg/ml, from about 0.5 µg/ml to about 20 µg/ml, from about 0.5 µg/ml to about 50, from about 0.5 µg/ml to about 100 µg/ml, from about 0.5 to about 200 µg/ml, from about 0.5 µg/ml to about 500 µg/ml, from about 15 µg/ml to about 25 µg/ml, from about 15 µg/ml to about 50, from about 18 µg/ml to about 30 µg/ml, from about 18 µg/ml to about 50 µg/ml.

The pharmaceutically acceptable compositions of this invention can be administered to humans and other animals orally, rectally, parenterally, intracisternally, intravaginally, intraperitoneally, topically (as by powders, ointments, or drops), bucally, as an oral or nasal spray, or the like, depending on the severity of the infection being treated. In certain embodiments, the compounds or the compositions of the invention may be administered orally or parenterally at dosage levels of about 0.01 mg/kg to about 50 mg/kg and preferably from about 1 mg/kg to about 25 mg/kg, of subject body weight per day, one or more times a day, to obtain the desired therapeutic effect.

In certain embodiments, the dosage amount of CFTR modulator in the dosage unit form is from 100 mg to 1,000 mg. In another embodiment, the dosage amount of CFTR modulator is from 200 mg to 900 mg. In another embodiment, the dosage amount of CFTR modulator is from 300 mg to 800 mg. In another embodiment, the dosage amount of CFTR modulator is from 400 mg to 700 mg. In another embodiment, the dosage amount of CFTR modulator is from 500 mg to 600 mg. The expression "dosage unit form" as used herein refers to a physically discrete unit of the CFTR modulator appropriate for the patient to be treated. It will be understood, however, that the total daily usage of the compounds and compositions of the present invention will be decided by the attending physician within the scope of sound medical judgment.

In another particular embodiment, the additional active compound is selected from a mucolytic agent, bronchodilator, an anti-biotic, an anti-viral agent, an anti-inflammatory agent, a CFTR modulator other than a compound of the present invention, a nutritional agent and combinations thereof.

In a particular embodiment the anti-viral agent is selected from one or more of the group consisting of an adenosine analog that inhibits viral replication *(Remdesvir),* a protease inhibitor *(Ritonavir- Nirmatrelvir),* a ribonucleoside *(Molnupiravir),* interferon beta, interferon alpha and combinations thereof.

Other additional active agents that can be used together with the CFTR modulators of the present invention are those disclosed in Rodriguez-Guerra M, Jadhav P, Vittorio TJ. Current treatment in COVID-19 disease: a rapid review. Drugs Context. 2021;10:2020-10-3. Published 2021 Jan 29. doi:10.7573/dic.2020-10-3; and García-Lledó A, Gómez-Pavón J, González Del Castillo J, et al. Pharmacological treatment of COVID-19: an opinion paper. Rev Esp Quimioter. 2022;35(2):115-130. doi:10.37201/req/158.2021.

In another embodiment, the additional active compounds is a compound selected from or more of the following: gentamicin, curcumin, cyclophosphamide, 4-phenylbutyrate, miglustat, felodipine, nimodipine, Philoxin B, geniestein, Apigenin, cAMP/cGMP modulators such as rolipram, sildenafil, milrinone, tadalafil, amrinone, isoproterenol, albuterol, and almeterol, deoxyspergualin, HSP 90 inhibitors, HSP 70 inhibitors, proteosome inhibitors such as epoxomicin, lactacystin, etc.

The amount of additional active compounds present in the compositions of this invention will be no more than the amount that would normally be administered in a composition comprising that therapeutic agent as the only active agent. Preferably the amount of additional therapeutic agent in the presently disclosed compositions will range from about 50% to 100% of the amount normally present in a composition comprising that agent as the only therapeutically active agent.

Liquid dosage forms for oral administration include, but are not limited to, pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof. Besides inert diluents, the oral compositions can also include adjuvants such as wetting agents, emulsifying and suspending agents, sweetening, flavoring, and perfuming agents.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic parenterally acceptable diluent or solvent, for example, as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

The injectable formulations can be sterilized, for example, by filtration through a bacterial-retaining filter, or by incorporating sterilizing agents in the form of sterile solid compositions which can be dissolved or dispersed in sterile water or other sterile injectable medium prior to use.

In order to prolong the effect of a compound of the present invention, it is often desirable to slow the absorption of the compound from subcutaneous or intramuscular injection. This may be accomplished by the use of a liquid suspension of crystalline or amorphous material with poor water solubility. The rate of absorption of the compound then depends upon its rate of dissolution that, in turn, may depend upon crystal size and crystalline form. Alternatively, delayed absorption of a parenterally administered compound form is accomplished by dissolving or suspending the compound in an oil vehicle. Injectable depot forms are made by forming microencapsule matrices of the compound in biodegradable polymers such as polylactide-polyglycolide. Depending upon the ratio of compound to polymer and the nature of the particular polymer employed, the rate of compound release can be controlled. Examples of other biodegradable polymers include poly(orthoesters) and poly(anhydrides). Depot injectable formulations are also prepared by entrapping the compound in liposomes or microemulsions that are compatible with body tissues.

Compositions for rectal or vaginal administration are preferably suppositories which can be prepared by mixing the compounds of this invention with suitable non-irritating excipients or carriers such as cocoa butter, polyethylene glycol or a suppository wax which are solid at ambient temperature but liquid at body temperature and therefore melt in the rectum or vaginal cavity and release the active compound.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidinone, sucrose, and acacia, c) humectants such as glycerol, d) disintegrating agents such as agar--agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like. The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes. Solid compositions of a similar type may also be employed as fillers in soft and hard-filled gelatin capsules using such excipients as lactose or milk sugar as well as high molecular weight polethylene glycols and the like.

The active compounds can also be in microencapsulated form with one or more excipients as noted above. The solid dosage forms of tablets, dragees, capsules, pills, and granules can be prepared with coatings and shells such as enteric coatings, release controlling coatings and other coatings well known in the pharmaceutical formulating art. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such a magnesium stearate and microcrystalline cellulose. In the case of capsules, tablets and pills, the dosage forms may also comprise buffering agents. They may optionally contain opacifying agents and can also be of a composition that they release the active ingredient(s) only, or preferentially, in a certain part of the intestinal tract, optionally, in a delayed manner. Examples of embedding compositions that can be used include polymeric substances and waxes.

Dosage forms for topical or transdermal administration of a compound of this invention include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulation, eardrops, and eye drops are also contemplated as being within the scope of this invention. Additionally, the present invention contemplates the use of transdermal patches, which have the added advantage of providing controlled delivery of a compound to the body. Such dosage forms are prepared by dissolving or dispensing the compound in the proper medium. Absorption enhancers can also be used to increase the flux of the compound across the skin. The rate can be controlled by either providing a rate controlling membrane or by dispersing the compound in a polymer matrix or gel.

By the term "effective amount" of a compound as provided herein is meant a nontoxic but sufficient amount of the compound to provide the desired result. As will be pointed out below, the exact amount required will vary from subject to subject, depending on the species, age, and general condition of the subject, the severity of the disease that is being treated, the particular compound used, its mode of administration, and the like. Thus, it is not possible to specify an exact "effective amount." However, an appropriate effective amount can be determined by one of ordinary skill in the art using only routine experimentation. In the context of compounds and compositions for use in the treatment of a subject suffering from betacoronavirus infection, the term "effective amount" of a compound as provided herein is meant a nontoxic but sufficient amount of the compound to treat betacoronavirus infection, for example: improvement in the infection or a symptom of the betacoronavirus, or lessening the severity of the infection or a symptom of the betacoronavirus.

A pharmaceutical composition may further comprise at least one pharmaceutically acceptable carrier. In some embodiments, the at least one pharmaceutically acceptable carrier is chosen from pharmaceutically acceptable vehicles and pharmaceutically acceptable adjuvants. In some embodiments, the at least one pharmaceutically acceptable is chosen from pharmaceutically acceptable fillers, disintegrants, surfactants, binders, lubricants.

It will also be appreciated that a pharmaceutical composition of this disclosure, including a pharmaceutical composition comprising combinations described previously, can be employed in combination therapies; that is, the compositions can be administered concurrently with, prior to, or subsequent to, at least one additional active pharmaceutical ingredient or medical procedures.

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1, about 2 and about 3"). When a listing of values is described the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in examples below.

In order that the invention described herein may be more fully understood, the following examples are set forth. It should be understood that these examples are for illustrative purposes only and are not to be construed as limiting this invention in any manner.

### Brief description of the figures

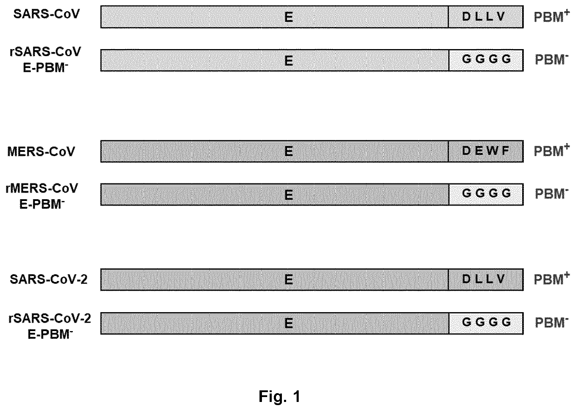

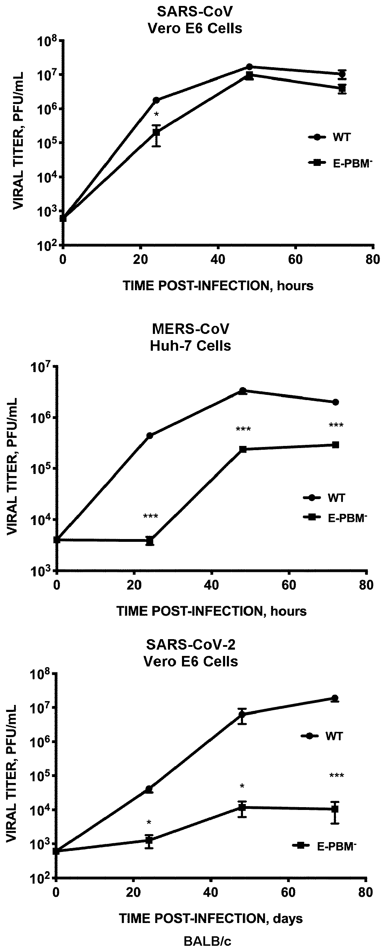

**Figure 1****. Generation of recombinant SARS-CoV, MERS-CoV and SARS-CoV-2 with the E PBM motif abrogated.** Schematic of mutants generated for each virulent hCoV. The enlarged genome regions represent the E protein. The last four amino acids of c-terminal end of E protein are schematized for the different viruses generated. SARS-CoV, MERS-CoV and SARS-CoV-2 viruses have the native E protein sequence. In the rSARS-CoV-E-PBM-, rMERS-CoV-E-PBM- and rSARS-CoV-2-E-PBM- mutants, the PBM domain of E protein of each virulent hCoV was replaced by four glycines. For each virus, whether or not it has the E protein PBM domain is indicated (PBM+ or PBM-, respectively).

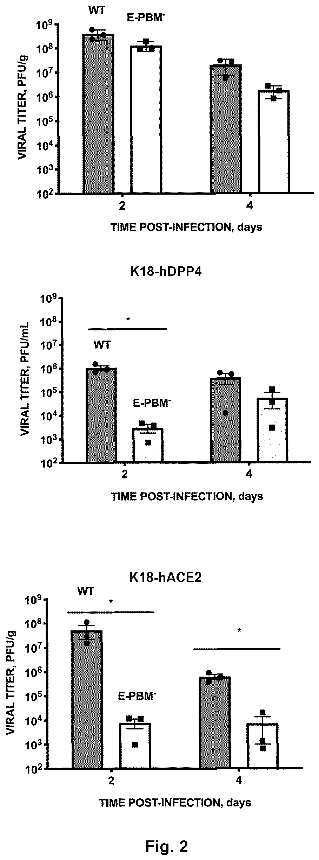

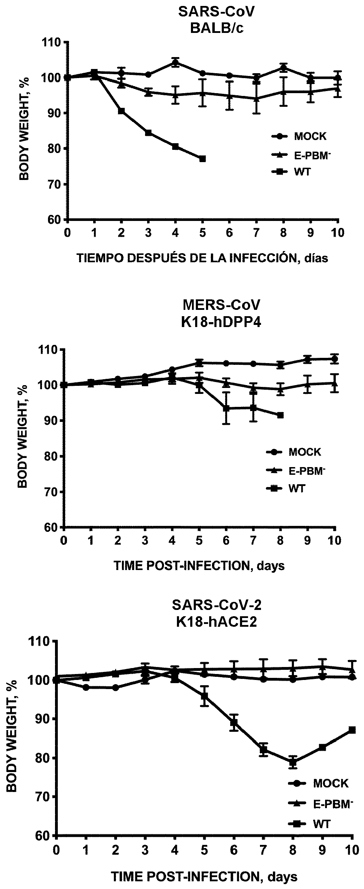

**Figure 2****. Growth kinetics of mutants lacking E protein PBM motif of SARS-CoV, MERS-CoV and SARS-CoV.** Subconfluent Vero E6, Huh-7 cells were infected with a MOI of 0.001 with SARS-CoV (top), SARS-CoV-2(bottom) or MERS-CoV (centre). The respective native viruses (wt) are shown with circle symbols and mutants without E protein PBM domain (PBM-) with square symbols. Supernatants were collected at 24, 48, and 72 hpi and titered by the lysis plate formation method. (B) Groups of 6 16-week-old Balb/c (top), K18-hDPP4 (centre), or K18-hACE2 (bottom) mice were inoculated intranasally with 10,000 ufp of the native viruses (gray columns) or each of their mutants lacking the PBM domain of E protein (PBM-) (white columns), respectively. Three mice from each group were sacrificed at 2 and 4 dpi to analyze virus production in lung. Vertical bars represent the standard error of the mean titers obtained by repeating the experiment three times. Statistically significant data are represented by asterisks according to the p-value obtained in Student's t-analysis: *, P value < 0.05; ***, P value <0.001.

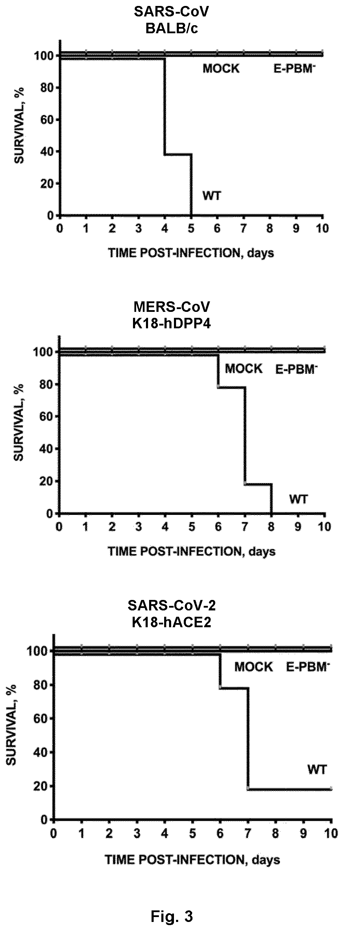

**Figure 3****. Virulence of recombinant viruses without E protein PBM motif: SARS-CoV, MERS-CoV, and SARS-CoV-2.** Groups of 5 16-week-old Balb/c, K18-hDPP4, or K18-hACE2 mice were inoculated intranasally with 10.000 PFUs with the parental (wt) SARS-CoV (top), MERS-CoV (centre), and SARS-CoV-2 (bottom) viruses (square symbol), or with their mutants without PBM domain of E protein (PBM-) (triangle symbol), respectively. Weight loss (three upper panels) and virulence (three lower panels) of mice were monitored for 10 days post infection. Vertical bars represent the standard error of the mean weight of the mice.

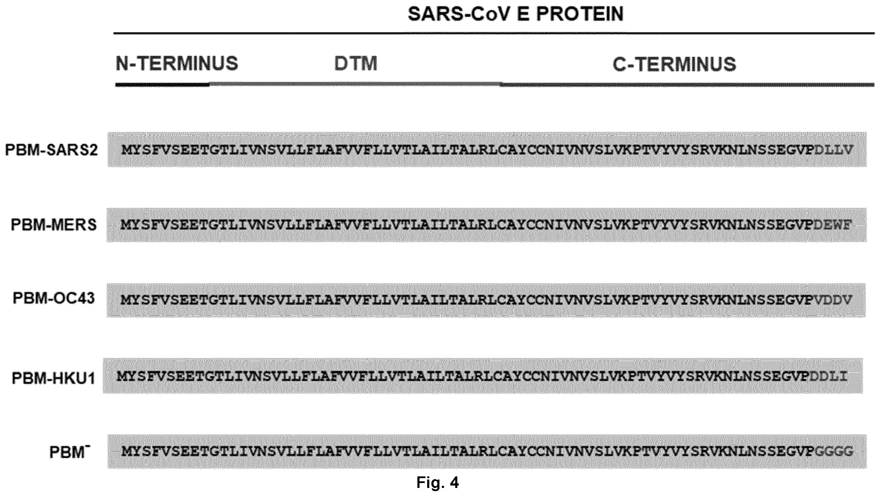

**Figure 4****. Recombinants of SARS-CoV with E protein PBM motifs from other** β-**hCoV** E protein sequence of parental virus and the respective mutants in which the PBM domain of E protein was replaced with that of another virulent or attenuated β-hCoV are represented in light gray.

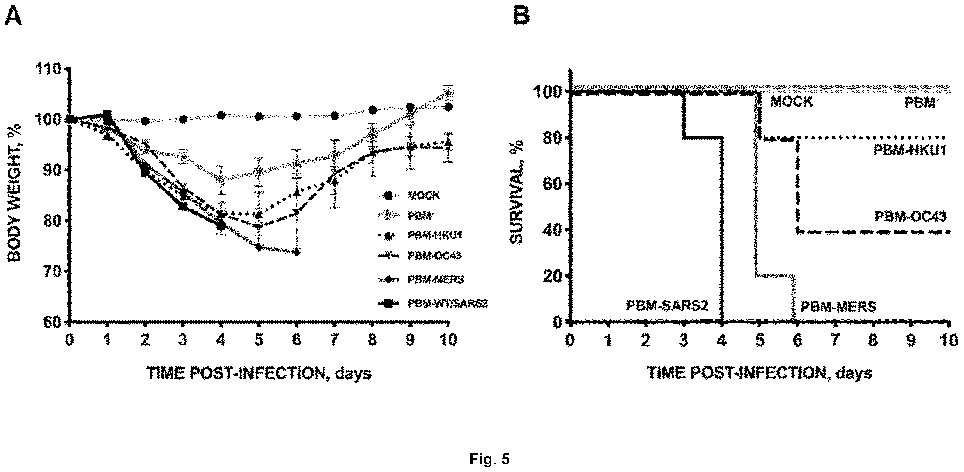

**Figure 5****. Virulence of SARS-CoV mutants with the PBM motif of E protein from attenuated orvirulent β-hCoV.** Groups of five 16-week-old Balb/c mice were inoculated intranasally with DMEM containing 100.000 PFUs of parental SARS-CoV (PBM-SARS-2), or with the generated mutants: PBM-MERS-CoV, PBM-hCoV-OC43, PBM-hCoV-HKU1, PBM-. Weight loss (A) and survival (B) were monitored for 10 dpi. Vertical bars represent the standard error of the mean weight of the mice.

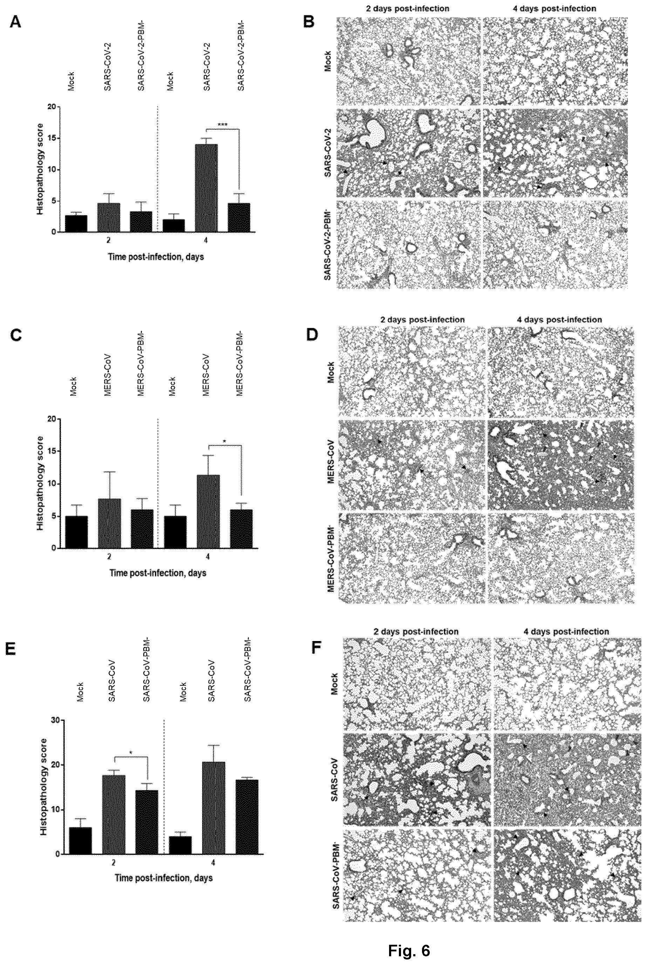

**Figure 6****. Lung pathology associated with E protein PBM of SARS-CoV, MERS-CoV and SARS-CoV-2.** Lung histopathology scores examined in lung samples taken from K18-hACE2 (A), K18-hDPP2 (C) and BALB/c mice (E) infected with SARS-CoV-2, MERS-CoV and SARS-CoV parental and mutant viruses, respectively as indicated in material and methods (n=3/group), and euthanized at 2 and 4 days post-infection (dpi). Representative lung histopathological sections (H&E staining) from K18-hACE2 (B), K18-hDPP4 (D) and BALB/c (F) mice euthanized at 2 and 4 dpi (magnification: 10x). Mean and SD of cumulative histopathological lesion scores. Unpaired t test: *p < 0.05; **p < 0.01; ***p < 0.001.

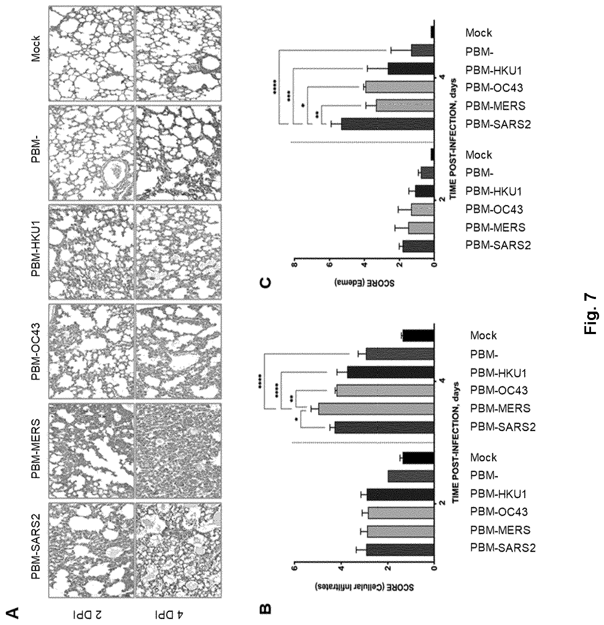

**Figure 7****. Lung pathology in SARS-CoV infected mice due to the presence of E protein PBM** from **attenuated or virulent β-hCoV.** Groups of six 16-week-old mice were mock infected or inoculated with 100.000 PFUs of PBM-SARS-2, PBM-MERS-CoV, PBM-hCoV-OC43, PBM-hCoV-HKU1, PBM⁻. Three mice from each group were sacrificed and lungs were collected at 2 and 4 dpi. (A) Lungs were fixed in formalin with zinc, embedded in kerosene, and cut and stained with hematoxylin and eosin. (B) Cellular infiltration and (C) edema scores examined in lung samples as indicated in material and methods (n=3/group). The vertical bars represent the standard deviation of the data obtained in the lung of each of the three mice used for each experimental variable. Unpaired t test: *p < 0.05; **p < 0.01; ***p < 0.001.

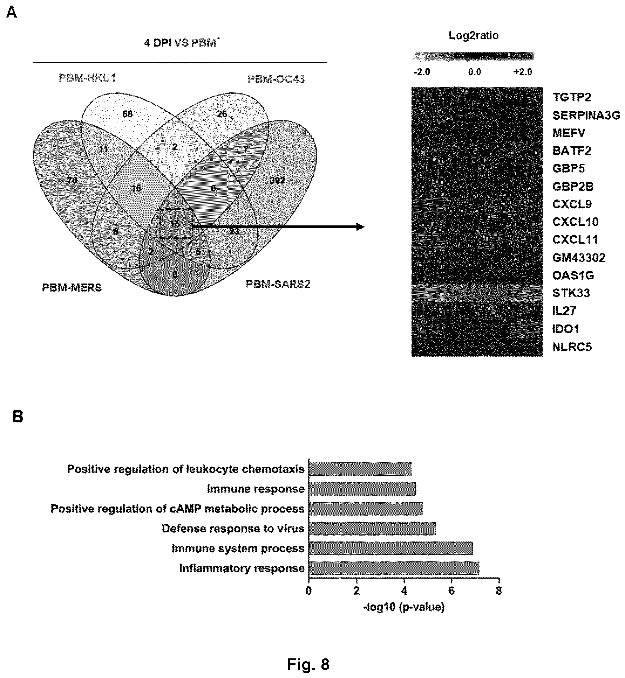

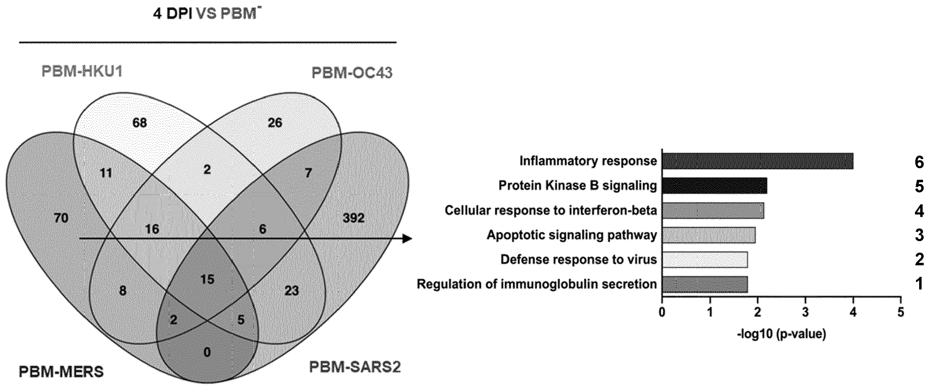

**Figure 8****. Differential and common gene expression patterns among the different rSARS-CoV with E protein PBM motif from virulent or attenuated β-hCoV.** (A) Venn diagram of genes differentially expressed at least 1.5-fold with a FOR less than 0.05 in the lungs of mice infected with the different rSARS-CoVs-E-PBM-[hCoV] compared to rSARS-CoV without E protein PBM domain at 4 dpi and the expression profile of the most frequently expressed genes due to the presence of a PBM motif from an attenuated or virulent hCoV in the SARS-CoV E protein. (B) Functional analysis of the 15 genes commonly expressed due to the presence of a functional E protein PBM. Clustering of the genes based on the biological process terms defined in Gene Ontology are depicted. The six most relevant biological processes at 4 dpi to which the 15 commonly expressed genes are shown. The values on the x-axis indicate the -Iog10 (p-value) values obtained with the DAVID software.

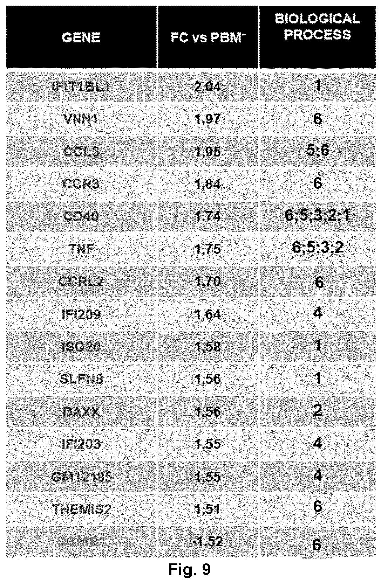

**Figure 9****. Biological activity groups of genes differentially expressed exclusively due to the presence of E protein PBM from MERS-CoV.** (A) Biological activity types into which the 70 genes that are differentially expressed in the lungs of rSARS-CoV-PBM-MERS-CoV-infected mice are grouped compared to rSARS-CoVs without PBM domain or with PBM motifs from other hCoVs. The grouping of these genes into distinct sets is based on the biological processes defined in Gene Ontology. The six most relevant biological processes are represented. (B) Table with information about the level of significance and the biological processes in which genes belonging to the six most relevant biological processes are involved. The names of the genes in dark gray and light gray correspond to those that increase or decrease their expression, respectively.

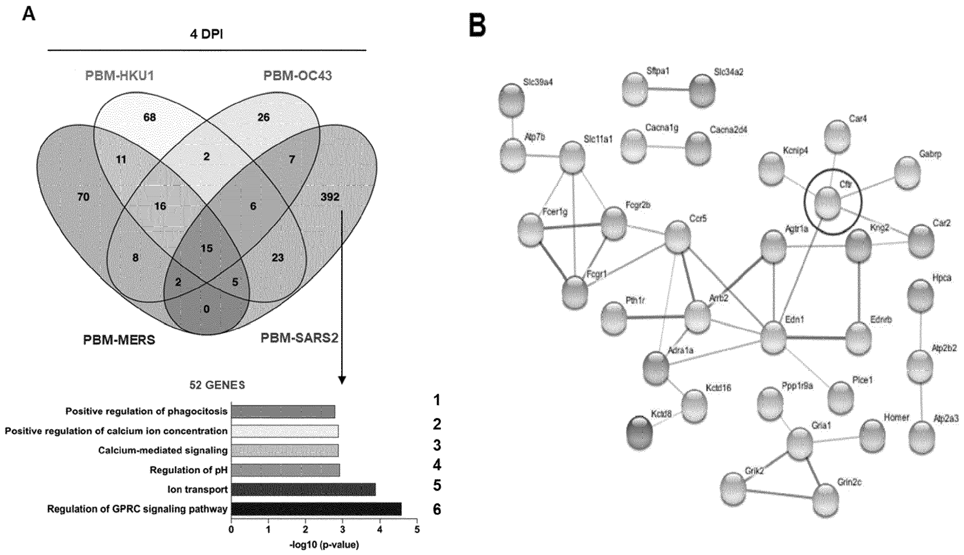

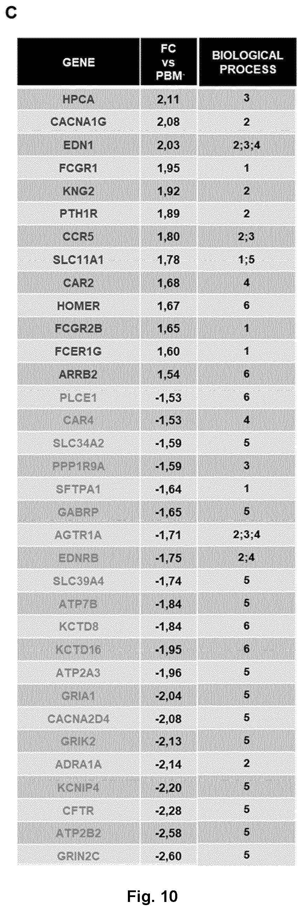

**Figure 10****. Biological activity groups of genes differentially expressed exclusively due to the presence of E protein PBM from SARS-CoV and SARS-CoV-2.** (A) Clustering of 392 genes that are differentially expressed in the lungs of mice infected with the native virus compared to rSARS-CoVs without a PBM domain or with a PBM motif from another hCoV. The clustering of these genes is based on the biological process terms defined in Gene Ontology and the 6 most relevant biological processes are represented. (B) Network of interactions between genes belonging to the most relevant biological processes using the STRING database. The thickness of the lines indicates the robustness of the data supporting each interaction and all those interactions with a score greater than 0.4 were considered valid. (C) Table with information on the rate of change and the biological processes to which the genes of the most relevant interaction networks belong. The names of the genes in dark gray correspond to those that increase their expression and in light gray to those that decrease it.

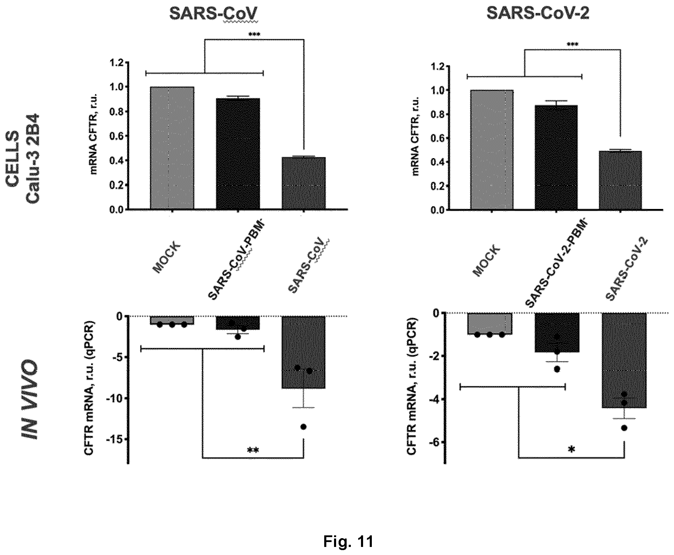

**Figure 11****. CFTR mRNA expression decreased during SARS-CoV and SARS-CoV-2 infection by E protein PBM.** CFTR mRNA expression Calu-3 cells and lungs from infected mice with SARS-CoV and SARS-CoV-2 with and without E protein PBM was analyzed by qPCR at 48 hpi and 4 dpi, respectively.

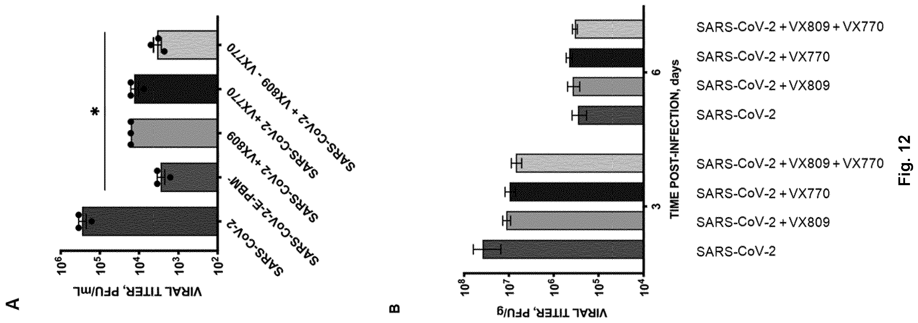

**Figure 12****. Effect of CFTR modulators on SARS-CoV-2 production.** (A) Subconfluent Calu-3 2B4 cells were infected with SARS-CoV-2 wt virus or with the mutant virus without E protein PBM at MOI of 0.1. Infected cells with the parental virus were incubated with Lumacaftor (VX809; 50uM), Ivacaftor (VX770, 5uM) or both. Culture supernatants were collected at 48 hpi and extracellular virus was titrated by the lysis plate formation method. (B) Groups of six 20-week-old K18-hACE2 mice were inoculated intranasally with 10.000 PFU and treated mice were inoculated intraperitoneally with Lumacaftor (12 mg/Kg) Ivacaftor (6 mg/Kg) or both daily until 8 dpi. Three mice from each group were sacrificed at 3 and 6 dpi to analyze virus production in lung. Vertical bars represent the standard error of the mean titers obtained by repeating the experiment three times. Statistically significant data are represented by asterisks according to the p-value obtained in Student's t-analysis: *, P value < 0.05.

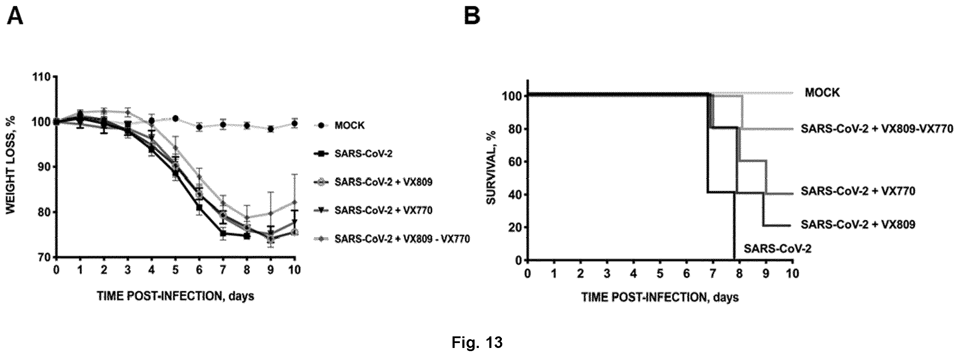

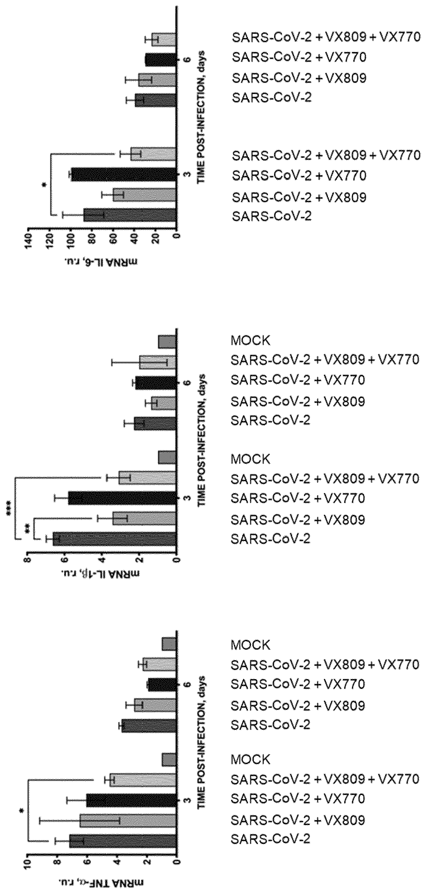

**Figure 13****. CFTR protection in mice infected with SARS-CoV-2.** Groups of five 20-week-old K18-hACE2 mice were mock infected or inoculated intranasally with 10.000 PFUs with the parental SARS-CoV-2 virus and treated once daily with Lumacaftor (12 mg/Kg) Ivacaftor (6 mg/Kg) or both until 8 dpi. Weight loss (A) and survival (B) of mice were monitored for 10 days post infection. Vertical bars represent the standard error of the mean weight of the mice.

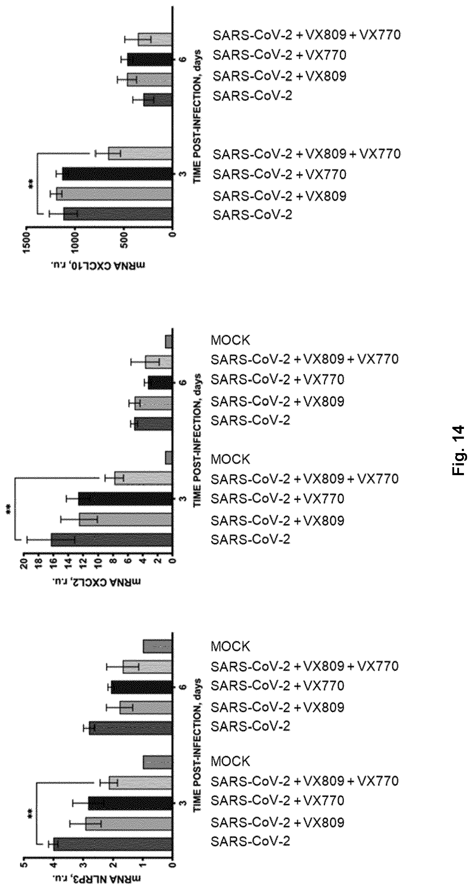

**Figure 14**. **Effect of CFTR modulators inhibition in the expression of inflammasome NLRP3 dependent cytokines and chemokines in SARS-CoV-2 infected mice.** mRNA quantification by RT-qPCR of the indicated inflammatory mediators at 3 and 6 dpi in the lungs of infected mice treated with Lumacaftor and Ivacaftor. mRNA levels were made relative to those in mock infected mice, using the ΔΔCt method and the 18s rRNA as the normalization endogenous control. Error bars indicate the standard error. Statistical significance was calculated by two-tailed Student's t test. p-value<0.05, **, p-value<0.01, ***, p-value<0.001.

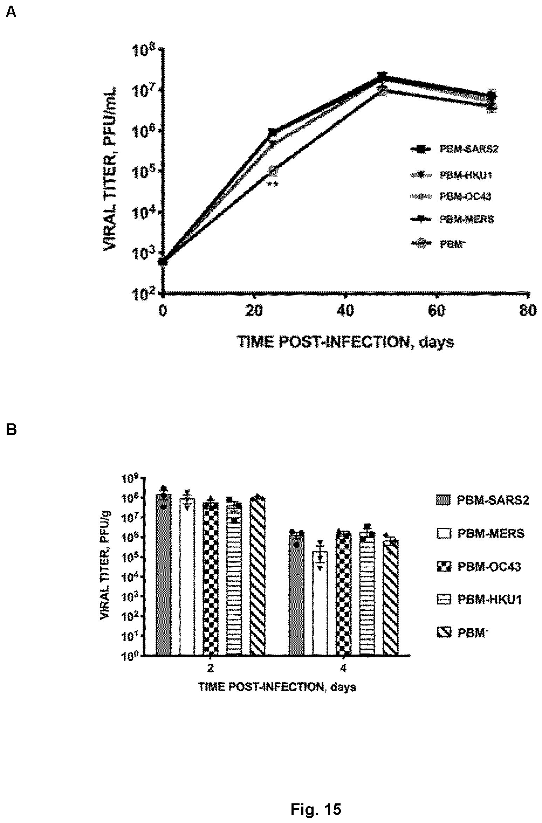

**Figure 15****. Growth kinetics of SARS-CoV E protein PBM mutants.** (A) Subconfluent Vero E6 with a MOI of 0.001 with SARS-CoV E protein PBM mutants. The respective native viruses (wt) and mutants without E protein PBM domain (PBM-), PBM-MERS-CoV, PBM.hCoV-OC43 and PBM-hCoV-HKU1. Supernatants were collected at 24, 48, and 72 hpi and titered by the lysis plate formation method. (B) Groups of six 16-week-old Balb/c mice were inoculated intranasally with 100.000 PFU of the native viruses or PBM-, PBM-MERS-CoV, PBM.hCoV-OC43 and PBM-hCoV-HKU1. Three mice from each group were sacrificed at 2 and 4 dpi to analyze virus production in lung. Vertical bars represent the standard error of the mean titers obtained by repeating the experiment three times. Statistically significant data are represented by asterisks according to the p-value obtained in Student's t-analysis: **, P value < 0.01.

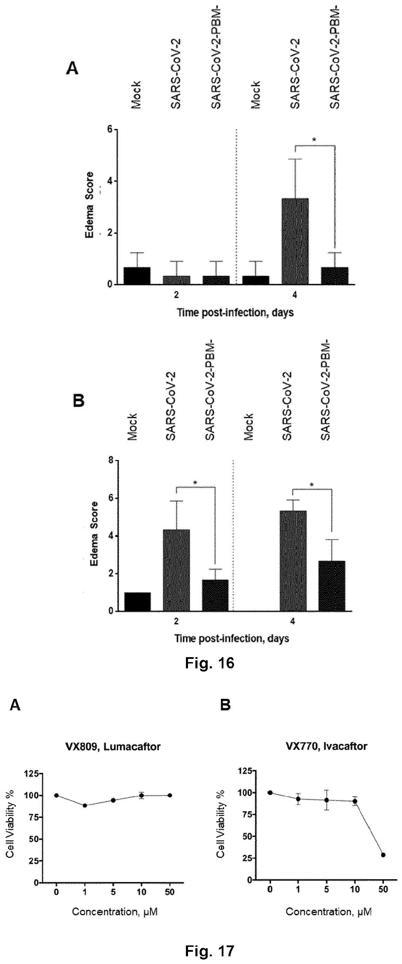

**Figure 16****. Pathology score in lung of mice infected with SARS-CoV and SARS-CoV-2 with and without E protein PBM.** Edema scores examined in lung samples taken from K18-hACE2 infected with SARS-CoV-2 parental and mutant viruses, respectively as indicated in material and methods (n=3/group) and euthanized at 2- and 4-days post-infection (dpi). Mean and SD of cumulative histopathological lesion scores. Unpaired t test: *p < 0.05; **p < 0.01; ***p < 0.001.
**Figure 17****. Viability of Calu-3 2B4 cells in the presence of different CFTR protein modulators.** Subconfluent and uninfected Calu-3 2B4 cells were incubated with different concentrations (1-50 µM) of Ivacaftor (A) and Lumacaftor (B). Supernatants were collected at 48 hdi and cell viability was determined by the MTT (3-(4,5-dimethylthiazol-2-yl)-2,5-diphenyltetrazole bromide) reduction assay. Mean values of cell viability and their standard deviations are shown.

### MATERIALS & METHODS

### Ethics statement

Animal experimental protocols were approved by the Environmental Council of Madrid (permit number: PROEX 112/14) and the Ethical Committee of the Center for Animal Health Research (CISA-INIA) (permit numbers: CBS 2014/005 and CEEA 2014/004) in strict accordance with Spanish National Royal Decree (RD 53/2013) and international EU guidelines 2010/63/UE about protection of animals used for experimentation and other scientific purposes and Spanish national law 32/2007 about animal welfare. All work with infected animals was performed in a biosafety level 3+ (BSL3+) laboratory of the Center for Animal Health Research (CISA-INIA, Madrid, Spain). Infected mice were housed in a self-contained ventilated rack (Allentown, NJ).

### Viruses

Mouse-adapted (MA15) (Roberts, A., et al., A mouse-adapted SARS-coronavirus causes disease and mortality in BALB/c mice. PLoS Pathog., 2007. 3: p. 23-37) parental wild-type (wt) and recombinant viruses were rescued from infectious cDNA clones generated in a bacterial artificial chromosome (BAC). rSARS-CoV-PBM- was generated as described previously. (Almazan, F., et al., Construction of a severe acute respiratory syndrome coronavirus infectious cDNA clone and a replicon to study coronavirus RNA synthesis. J. Virol., 2006. 80: p. 10900-10906. Fett, C., et al., Complete protection against severe acute respiratory syndrome coronavirus-mediated lethal respiratory disease in aged mice by immunization with a mouse-adapted virus lacking E protein. J. Virol., 2013. 87: p. 6551-6559. DeDiego, M.L., et al., Pathogenicity of severe acute respiratory coronavirus deletion mutants in hACE-2 transgenic mice. Virology, 2008. 376: p. 379-389. Regla-Nava, J.A., et al., Severe acute respiratory syndrome coronaviruses with mutations in the E protein are attenuated and promising vaccine candidates. J. Virol., 2015. 89: p. 3870-3887.).

### Generation of recombinant virus infectious clones

The cDNA of SARS-CoV-MA15 assembled in a pBAC (pBAC-SARS-CoV-MA15), was used to introduce the corresponding mutations affecting E protein of SARS-CoV. To generate viruses that affect the carboxyl terminal of the E protein of SARS-CoV, DNA fragments that included nucleotides 26017 to 26884 of SARS-CoV-MA15 genome flanked by *BamHI* and *Xcml* restriction sites were generated using the oligos indicated in **Table 1.** These fragments carried different mutations located in the E gene that generated amino acid changes at carboxy terminus of E protein: PBM-MERS, PBM-HKU1, PBM-OC43. The fragments were digested with the enzymes *BamHI* and *Xcml* and introduced into an intermediate plasmid pBAC-*BamHI-RsrII*-SARS-CoV containing nucleotides 26044 to 29783 of the infective clone of SARS-CoV. Next, these plasmids were digested with the enzymes *BamHI* and *RsrII,* and the fragments containing the different mutations were inserted into the plasmid pBAC-SARS-CoV-MA15 digested with the same enzymes, obtaining the respective infective clones. The integrity of cDNA was verified by restriction analysis with the enzyme *HindIII* and sequencing.

**Table 1: Oligonucleotides used**

| Virus | Primer | SEQ ID N° | Sequence (5'-> 3') |
|---|---|---|---|
| **PBM-MERS** | SARS-E-VS | SEQ ID NO 3 | CTCTTCAGGAGTTGCTAATCCAGC AATGG |
| | SARS-E-PBM-MERS-RS | SEQ ID NO 4 | TTAGAACCATTCATCAGGAACTCCT TCAGAAGAGTTCAG |
| | SARS-E-PBM-MERS-VS | SEQ ID NO 5 | TTAGAACCATTCATCAGGAACTCCT TCAGAAGAGTTCAG |
| | SARS-26885-RS | SEQ ID NO 6 | GGTCCTTAATGTCACAGCGCCC |
| **PBM-HKU1** | SARS-E-VS | SEQ ID NO 7 | CTCTTCAGGAGTTGCTAATCCAGC AATGG |
| | SARS-E-PBM-HKU1-RS | SEQ ID NO 8 | CGTTTAGATCAGATCATCAGGAAC TCCTTCAGAAGAGTT |
| | SARS-E-PBM-HKU1-VS | SEQ ID NO 9 | GAAGGAGTTCCTGATGATCTGATC TAAACGAACTAACTATTA |
| | SARS-26885-RS | SEQ ID NO 10 | GGTCCTTAATGTCACAGCGCCC |
| **PBM-OC43** | SARS-E-VS | SEQ ID NO 11 | CTCTTCAGGAGTTGCTAATCCAGC AATGG |
| | SARS-E-PBM-OC43-RS | SEQ ID NO 12 | TTAGACGTCATCAACAGGAACTCC TTCAGAAGAG |
| | SARS-E-PBM-OC43-VS | SEQ ID NO 13 | TCTGAAGGAGTTCCTGTTGATGAC GTCTAAACGAACTAACTATTA |
| | SARS-26885-RS | SEQ ID NO 14 | GGTCCTTAATGTCACAGCGCCC |
| **MERS-CoV-PBM** | SA27502VS | SEQ ID NO 15 | GCTTATCGTTTAAGCAGCTC |
| | Rs mutPBM | SEQ ID NO 16 | CATATTAGACATTATGAAGGAGTTC GTTATCCTCCTCCTCCAGGTGGT |
| | VS mutPBM | SEQ ID NO 17 | AACCCCCTCTACCACCTGGAGGAG GAGGATAACGAACTCCTTCATAAT |
| | SA28319RS | SEQ ID NO 18 | TCTGTCGTAGTCACAAGCAC |

SARS-CoV-2 cDNA assembled in the pBAC (pBAC-SARS-CoV-2) was used to introduce the corresponding mutations affecting the PBM motif of the SARS-CoV-2 E protein. First, a DNA fragment was obtained by chemical synthesis (GeneScript) that included nucleotides from 26386 to 26751 of SARS-CoV-2 genome, flanked by the *Hpal* and *Agel* restriction sites. This fragment carried the mutation located in the carboxy terminus, eliminating the PBM motif (DLLV -> GGGG). The fragment containing these mutations was introduced into the plasmid psL-F6-SARS-CoV-2, which contains nucleotides 25314-28610 of the SARS-CoV-2 genome, with previously described restriction enzymes, generating plasmid psL- F6-SARS-CoV-2-E-PBM-. Plasmid psL-F6-SARS-CoV-2-E-PBM- was then digested with restriction enzymes *BamHI* and *AvrII* and cloned into an intermediate plasmid pBAC-F6-SARS-CoV-2. Subsequently, this plasmid with the unique *BamHI* and *RsrII* cloning sites was digested and the fragment containing the E protein gene mutation was inserted into the pBAC-SARS-CoV-2 plasmid digested with the same enzymes to generate the corresponding cDNA infectious clone. The integrity of the cloned DNA was verified by restriction analysis with the enzyme *EcoRI* and by sequencing.

The cDNA infectious clone of MERS-CoV strain EMC/2012, assembled in the pBAC (pBAC-SA-FL), was used to generate the mutants that modified the PBM motif of the E protein of MERS -CoV. First, DNA fragments including nucleotides 27502 to 28319 of MERS-CoV genome flanked by *KfII* and *PfI23II* restriction sites were generated using the oligos indicated in Table 1. The fragment obtained was introduced into an intermediate plasmid pBAC-SA-F6 including nucleotides 25841-30162 of MERS-CoV genome using the restriction enzymes *KfII* and *PfI23II.* The 25841-30162 region of MERS-CoV genome is flanked by the *PacI* and *RsrII* restriction sites in the plasmids pBAC-SA-FL and pBAC-SA-F6. The *PacI-RsrII* region of intermediate plasmid pBAC-SA-F6, with the desired E protein gene mutation was cloned into pBAC-SA-FL to generate the complete infective cDNA of the E protein PBM mutant. All cloning steps were verified by sequencing.

### Recovery of recombinant virus variants from the cDNA clones

Vero E6, Huh-7 and Vero E6-TMPRSS2 cells were grown to 95%confluence in 12.5 cm2 flasks and transfected with 6 µg of infectious cDNA clone and 18 µl of Lipofectamine 2000 (Invitrogen), according to the manufacturer's specifications for SARS-CoV, MERS-CoV and SARS-CoV, respectively. At 6 hours post transfection (hpt) the medium was replace and incubated at 37 °C for 72 h. Cell supernatants were harvested and passaged once on fresh cells, and the recovered viruses were cloned by three rounds of plaque purification following standard procedures for SARS-CoV. In the case of MERS-CoV and SARS-CoV-2, at 72 hours the supernatants were collected and titrated by lysis plaques in semi-solid medium. Viruses were then amplified in 75 cm² flasks, using monolayers of the cells indicated for each virus, infecting cells with a 0.001 moi. The supernatants were collected at 72h and titrated. The genome of 3' end of different viruses was sequenced, from the end of the replicase to the 3'UTR of each virus. All sequences were compared to the parental wild-type (wt) virus sequence using SeqMan software (Lasergene, Madison, Wl).

### Growth kinetics

Subconfluent monolayers (around 90% confluency) of Vero E6 and Huh-7 cells were infected at a MOI of 0.001 with parental viruses or the respective mutant virus without E protein PBM. Culture supernatants were collected at 24, 48 and 72 hpi and virus titer were determined as previously described (DeDiego, M.L., et al., A severe acute respiratory syndrome coronavirus that lacks the E gene is attenuated in vitro and in vivo. J. Virol., 2007. 81: p. 1701-1713.).

### Mice

For the pathogenesis experiments of SARS-CoV, MERS-CoV and SARS-CoV-2 viruses, 16-week-old BALB/c OlaHsd (Harlan), K18-hDPP4 (Zhao et al., 2014) and K18-hACE2 (Jackson) were used, respectively. Mice were anesthetized with isoflurane and inoculated intranasally using 100.000 or 10.000 PFUs of the corresponding virus in a 50 µl volume of DMEM supplemented with 2% FBS. Manipulation of infected mice was carried out in a level 3+ biological containment laboratory at CISA-INIA (Algete, Madrid) equipped with the containment infrastructure described above for working with cell cultures and, in addition, using a ventilated Animal rack transport unit-Bio containment unit (Harvard) to maintain the mice during the development of the experiment. Virus titrations were performed as previously described (DeDiego, 2007). Viral titers were expressed as PFU counts per gram of tissue.

### Lung viral titers of infected mice

Lungs from infected mice were harvested and weighed at different days post infection (dpi). Lungs were homogenized in 2 mL of PBS containing 100 IU/mL penicillin, 0.1 mg/ml streptomycin, 50 µg/ml gentamicin, and 0.5 µg/ml amphotericin B (Fungizone), using gentleMACS Dissociator (Miltenyibiotec). Virus titer were determined as previously described (DeDiego, 2007).

### Lung Histopathology

The entire left lung lobe was removed from each mouse and immersion-fixed in zinc formalin (Sigma-Aldrich) for 48h. After fixation period, samples were routinely processed and embedded in paraffin blocks that were then sectioned at 4 µm thickness on a microtome, mounted onto glass slides and routinely stained with haematoxylin and eosin (H&E). Lung sections were microscopically evaluated using an Olympus BX43 microscope by a single veterinary pathologist who was blinded to the identity of mice (Veterinary Pathology Department, Animal Health Research Center-CISA, Valdeolmos, Spain). To assess the presence and severity of histopathological lesions, lung inflammation parameters based on previous reports on SARS-CoV-2 infection in mouse models were used (Sun, J., et al., Generation of a Broadly Useful Model for COVID-19 Pathogenesis, Vaccination, and Treatment. Cell, 2020. 182: p. 734-743 e5). The histopathological parameters evaluated were the follows: alveolar haemorrhages; alveolar oedema; perivascular oedema; alveolar septal thickening (interstitial pneumonia); inflammatory cell infiltration in alveoli; bronchi/bronchioles with epithelial necrosis, detached epithelium or inflammatory cells in the lumen (bronchitis/bronchiolitis); peribronchial/peribronchiolar and perivascular mononuclear infiltrates; cytopathic effect in pneumocytes or syncytia. The histopathological parameters were graded following a semi-quantitative scoring system as follows: (0) no lesion; (1) minimal lesion; (2) mild lesion; (3) moderate lesion; (4) severe lesion. The cumulative scores of histopathological lesions provided the total score per animal. In each experimental group, the individual scores were used to calculate the group average. To assess differences between experimental groups, unpaired t-test was used for statistical analysis of histopathological scores. The statistical significances are indicated as follows: *p < 0.05; **p < 0.01; ***p < 0.001.

### Extraction and analysis of RNA

Cell culture or lung RNA samples were collected and incubated in RNAlater reagent (Ambion). To extract total RNA, lungs were homogenized in 2 ml of RLT lysis buffer (QIAGEN) supplemented with 1% β-mercaptoethanol using the GentleMACS Dissociator system (Miltenyi Biotec) following the protocol proposed by the manufacturer. RNA was purified from supernatants using the RNeasy mini kit reagent (QIAGEN) as previously described. To generate cDNAs from the purified RNAs, a reverse transcription (RT) reaction was carried out using the High Capacity cDNA RT kit reagent (Applied biosystems). To verify the sequence of the mutations introduced into the viral genome, 4 µl of the cDNA generated in the RT reaction was used as a template in a PCR reaction using the enzyme Taq Polymerase (Invitrogen). Cellular mRNA expression was analyzed by quantitative RT-PCR (RT-qPCR), using TaqMan^{®} technology with commercial probes (ThermoFisher Scientific) (Table 2). In all cases, the reaction was performed with the FastStart Universal Probe Master reagent (Rox) (Roche). qPCR reactions were performed in a 7500 Real Time PCR System (ThermoFisher Scientific. qPCR data was analyzed using the 7500 software v2.0.6 (ThermoFisher Scientific). All experiments met the recommendations for analysis of gene expression by RT-qPCR (MIQE) (Bustin et al., 2009). Each result is the average of three independent experiments in which each sample was tested in triplicate. The relative quantification of gene expression was performed from the mean values of Ct (cycle in which the amplification curve cuts at the threshold level using the 2-ΔΔCt method (Livak and Schmittgen, 2001). The level of the 18s ribosomal RNA (18s rRNA) was used as an endogenous control to normalize cellular mRNA levels in mouse lungs, while hydroxymethylbilane synthase (HMBS) was used to normalize cellular mRNA levels in cell cultures.

**Table 2: Probes used in the present invention**

| Gene | Probe (Thermo Fisher) |
|---|---|
| mNLRP3 | Mm00840904_m1 |
| mTNF-α | Mm00443258_m1 |
| mIL-6 | Mm00446190_m1 |
| mIL-1β | Mm01336189_m1 |
| mCXCL10 | Mm00445235_m1 |
| mCXCL2 | Mm00436450_m1 |
| 18s rRNA | Mm03928990_g1 |
| HMBS | Hs00609297_m1 |

### Deep sequencing.

Total RNA extracted from lungs of infected mice was used for RNaseq sequencing. Novogene company (Shanghai) digested the samples with the enzyme DNAsal (Roche) to eliminate possible DNA contamination. RNA was processed to remove rRNA, a major component in large RNA samples, and enrich the resulting sequences for less abundant expressed RNAs such as mRNAs. RNA libraries were constructed (Illumina method) and 300 nt single-end reads were obtained in which the complementary strand was marked in order to distinguish the original sense of the sequences. Three replicates per variable and per day were sequenced. From each sample, 80 million sequences were obtained.

### Genomic alignment of Illumina paired-end reads

Paired-end reads in FASTQ format were quality-checked with FASTQC (www.bioinformatics.babraham.ac.uk/projects/fastqc; 2021). As no limitations were detected for each sample, all unprocessed reads were aligned to mouse genome (GRCm38, primary assembly) using RNA-STAR (--alignlntronMax 1000000 - alignMatesGapMax 1000000). Duplicated reads, i.e., those aligned at identical genomic positions) were marked using MarkDuplicates function of picard-tools (https://broadinstitute.github.io/picard/; 2021). Alignment files, in SAM format were compressed, sorted and indexed with corresponding commands of Samtools package (Danecek, P., et al., Twelve years of SAMtools and BCFtools. Gigascience, 2021.10(2)). IGV *(Integrative genomics viewer)* browser was used to visualize alignment results.

### Quantification of mouse genes and differential expression determination

All mouse genes (ENSEMBL release 101) were quantified using featureCounts function of bioconductor package Rsubread (Liao, Y., G.K. Smyth, and W. Shi, The R package Rsubread is easier, faster, cheaper and better for alignment and quantification of RNA sequencing reads. Nucleic. Acids Res., 2019. 47: p. e47.) (ignoreDup=TRUE, requiredBothEndsMapped=TRUE, primaryOnly=TRUE). To calculate differential expression between pairs of replicated samples, DESeq and IfcShrink functions of bioconductor package DESeq2 were used (cooksCutoff=FALSE, independentFiltering=TRUE, type="normal"). Differential expression results, including adjusted p-values by FDR, were stored as text-tabulated files and converted to FIESTA format (https://bioinfogp.cnb.csic.es/tools/FIESTA; 2021) to facilitate the visualization of over-expressed and under-expressed genes in form of MA plots. For each comparison, genes with IlogRatiol >= 1 and FOR <= 0.05 were considered differentially expressed.

### Functional analysis of relevant genes

DAVID tool (Sherman, B.T., et al., DAVID: a web server for functional enrichment analysis and functional annotation of gene lists (2021 update). Nucleic Acids Res, 2022.) was used to determine over-representation of Gene Ontology terms (Biological Process) in differentially expressed genes (both, over- and under-expressed). The six most representative biological process were selected based on the p-value.

### Mice antiviral experiment

Mice were treated by intraperitoneal (i.p.) injection administration with an appropriate concentration of compounds 24 hours prior to infection with 10.000 PFUs of SARS-CoV-2. Mock infected or infected animals were treated once daily with 12 or 6 mg/Kg of Lumacaftor or Ivacaftor, respectively. All treatments ceased after day 8 post infection, and animals were sacrificed at days 3 and 6 dpi.

### Cell viability assay

To determine the cytotoxicity of commercial compounds that modulate CFTR protein activity, cells were incubated with different concentrations (1-50 µM) of Ivacaftor (VX-770, Merck) or Lumacaftor (VX-809, Selleck Chemicals) and cell viability was determined by the MTT (3-(4,5-dimethylthiazol-2-yl)-2,5-diphenyltetrazol bromide) reduction assay.

### Statistical analysis

Two-tailed, unpaired Student's t tests were used to analyze the differences in mean values between groups.

### Examples

### Relevance of E protein PBM from virulent human coronaviruses (hCoVs) on viral replication in vitro and in vivo

To study the implication of E protein PBM for virulent hCoVs replication *in vitro* and *in vivo,* a collection of mutant viruses in which E protein PBM function was deleted by replacing the 4 residues of the PBM core by glycine was engineered using an infectious cDNA clone (Fig. 1). The growth kinetics of SARS-CoV and SARS-CoV-2 wild type and deletion mutants in Vero E6 and, in the case of MERS-CoV using Huh-7 was determined.

SARS-CoV-2 and MERS-CoV E PBM deletion mutants showed between 10 and 1000-fold lower viral titers, respectively, than the parental (wt) viruses (Fig 2 upper panels), in contrast with the rSARS-CoV without E PBM, indicating that E PBM was required for optimal virus replication in cell culture for both SARS-CoV-2 and MERS-CoV.

To determine the relevance of E protein PBM in *in vivo* virus growth, BALB/c, K18-hDPP4 or K18-hACE2 mice were infected either with SARS-CoV, MERS-CoV, and SARS-CoV-2 wt viruses, or with each of the corresponding deletion mutants without the E PBM motif, respectively, and viral titers in lung were determined at days 2 and 4 post-infection (dpi) (Fig. 2 bottom panels). MERS-CoV and SARS-CoV-2 E protein deletion mutants grew 1000- to 10000-fold lower titers that the corresponding parental viruses, indicating that the corresponding E protein PBMs were relevant for the efficient replication of this viruses both *in vitro* and *in vivo.*

### E protein PBM is a virulence factor in virulent hCo V

To evaluate the role E PBM motif in hCoV pathogenesis, BALB/c, K18-hDPP4 or K18-hACE2 mice were either mock infected, or infected with parental SARS-CoV, MERS-CoV and SARS-CoV, or with the corresponding E PBM motif deletion mutants, respectively. Loss weigh and survival were monitored through 10 dpi. All mice infected with parental viruses lost weight and between 80 and 100% of them died (Fig. 3). In contrast, all mice infected with highly virulent recombinant virus without E PBM motif survived. There was no difference between this group and the mock group. These results indicate that E protein PBM is a virulence factor in virulent hCoV.

To study the influence of PBM motifs from attenuated or virulent hCoVs, a set of recombinant SARS-CoVs with E protein PBM sequence substituted by those of the virulent or attenuated β-hCoV was engineered including the PBM from SARS-CoV-2, MERS-CoV, and SARS-CoV-HKU1 or HCoV-OC43) (Fig 4). As a control, a rSARS-CoV without a PBM core sequence was generated (PBM⁻). To analyze their pathogenicity, BALB/c mice were intranasally inoculated with recombinant viruses, and weight losses (Fig 5A) and survival (Fig 5B) were monitored for 10 dpi. All mock-infected mice and those infected with the virus lacking E protein PBM survived. In contrast, mice infected with recombinant viruses with the parental virus PBM, from SARS-CoV-2 (PBM-SARS2), or with E protein PBM core from MERS-CoV (PBM-MERS) lost weight and all of them died between 4 and 6 dpi respectively. Interestingly, a gradient of lethality among these highly virulent viruses was observed. In fact, all mice infected with PBM-SARS2 died even before losing 20% of weight. In contrast all mice infected with PBM-MERS lost more than 25% of weight and were sacrificed. Mice infected with viruses that have E protein PBM core sequence from attenuated hCoVs, such as PBM-OC43 and PBM-HKU1, showed intermediate survival phenotypes, and 40% and 80%, respectively, of infected mice survived. These results indicated that changing the PBM core sequence of E protein by a functional PBM core present in another hCoVs changed the virulence pattern of SARS-CoV. Viral titers in lung of mice infected with SARS-CoVs including different PBMs were similar, indicating that differences in SARS-CoV-E-PBM mutants virulence were not due to changes in viral titers (Fig. 15). The presence in SARS-CoV of its native PBM domain, which is the same as that of SARS-CoV-2, caused the highest pulmonary edema and lethality. However, the replacement of the PBM core by that of MERS-CoV PBM led to an increase in cellular infiltrates and thickening of the alveolar walls. These results demonstrate that both PBM motifs were associated to a high virus pathogenicity virulence.

### E protein PBM motif conditions β-hCoV pathogenesis

K18-hACE2 mice infected with SARS-CoV-2 displayed mild thickening of the alveolar septae along with mild perivascular and peribronchiolar mononuclear infiltrates. At day 4 dpi, such lesions were increased in mice infected with SARS-CoV-2, also showing many areas with alveolar oedema (Fig 6A-B; Fig 17). However, mice infected with the mutant SARS-CoV-2-PBM⁻ remained without displaying significant inflammatory and vascular changes. K18-hDPP4 mice infected with MERS-CoV showed moderate lung inflammatory lesions at day 2 dpi that increased in severity at day 4 dpi, highlighting the presence of diffuse thickening of the alveolar septae, moderate perivascular and peribronchiolar mononuclear infiltrates. On the contrary, mice infected with the mutant MERS-CoV-PBM⁻, inflammatory lesions were not significant at any time (Fig 6C-D). Lung inflammatory lesions were more severe in BALB/c mice infected with SARS-CoV than in BALB/c mice infected with the mutant SARS-CoV-PBM⁻. Among them stood out the presence of severe interstitial pneumonia, severe perivascular and peribronchiolar mononuclear infiltrates and severe alveolar oedema (Fig 6E-F).

Lung sections from mice infected with SARS-CoVs including PBM motifs from either attenuated or virulent CoVs, collected at 2 or 4 dpi were stained with hematoxylin and eosin. Histological examination of mock-infected mice showed clean and functional airways during the whole experiment. Mice infected with virus lacking E protein PBM core sequence (PBM⁻) showed minimal inflammatory cell infiltrates and edema only at 4 dpi (Fig. 7). Whereas lung sections of mice infected with rSARS-CoV with an E protein PBM from either HKU1-CoV (PBM-HKU1) or OC43-CoV (PBM-OC43) showed significant infiltrates at 2 dpi, which didn't increase at 4 dpi, in contrast with that of mice infected with virulent SARS-CoVs (Fig. 7). Mice infected with viruses including an E protein PBM core from SARS-CoV-2 showed moderate interstitial and peribronchiolar cell infiltration at 2 dpi, and a higher lung edema accumulation at 4 dpi was observed, associated with the higher lethality of this virus. Interestingly, mice infected with recombinant viruses including an E protein PBM core from MERS-CoV showed at 2 dpi a phenotype similar to that observed for PBM-SARS2, but with a significant increase in the infiltration and alveolar and bronchiolar wall thickening at 4 dpi, in comparison with those from mice infected virus mutants including alternative E PBM motifs from attenuated CoVs (Fig. 7). These data demonstrate that differences in virus virulence correlated with strong lung pathology and, and that the sequence of E protein PBM core sequence is responsible for this observation.

### Effect of E protein PBM motif virus origin on host gene expression

To determine the virulence mechanism of rSARS-CoV with PBM motifs from hCoVs with different virulence, a deep RNAseq analysis was performed in lungs from infected mice, at 2 and 4 dpi, but this study was focused at 4 dpi, the time point at which higher differences in lethality, virulence and lung damage were observed A Venn's Diagram of differentially expressed genes (|FC|>1.5; FDR<0.05), in the lungs of mice infected with rSARS-CoVs with PBMs from hCoVs causing different levels of pathogenicity, in comparation to PBM⁻ mutants, was displayed. The objective was to identify genes with significantly altered expression exclusively due to the presence of an E protein PBM from either attenuated or virulent β-hCoV. The presence of E protein PBM from attenuated β-hCoV reduced SARS-CoV mortality, and a set of 14 genes with up-regulated expression was identified, associated to the infection with virus mutants including E PBM motifs either from attenuated or virulent β-hCoV (Fig. 8A). These genes were classified in biological processes mainly related to immune and inflammatory responses, based on the Gene Ontology analysis (DAVID software) (Fig. 8B), indicating that during SARS-CoV infection, the presence of a functional E protein PBM motif from either an attenuated or virulent β-hCoV triggered a host innate immune response, in comparison with the immune response elicited by hCoVs without a PBM motif in the carboxyl terminal of E protein.

The replacement of SARS-CoV E protein PBM core by that from the homologous sequence of a virulent hCoV (PBM-MERS or PBM-SARS-2), increased lung damage and virus pathogenicity in comparison with the replacement by the PBM core from attenuated hCoV (PBM-HKU1 and PBM-OC43). The experiment focused on the 70 and 392 differentially expressed genes exclusively due to the presence of the E protein PBM from MERS-CoV or SARS-CoV-2, respectively at 4 dpi (Fig. 9A). The 70 differentially expressed genes, showing the highest difference level, exclusively due to the replacement of the native PBM by the homologous motif of MERS-CoV were related to activity groups associated to the immune and inflammatory responses (Fig. 9A-B). These changes were associated with higher cell infiltration and wall thickening observed in lungs infected with CoVs including a PBM-MERS. The observed infiltration levels were even higher that those observed when the PBM was replaced by the homologous motif from SARS-CoV-2 (PBM-SARS2). High levels of SLFN8, CCL3, CCR3, and CCRL2 were observed in addition to factors related to T-cell proliferation, leukocyte recruitment, eosinophil accumulation, and recruitment of effector immune cells, respectively.

From the 392 differentially expressed genes due exclusively to the presence of E PBM motif from SARS-CoV or SARS-CoV-2, 52 were included within the most statistically significant biological activity processes related to ion transport and homeostasis (Fig. 10B). At least 34 of these 52 genes were identified in the most relevant interaction network (Fig. 10C). The up-regulated genes were associated mainly to the regulation of cytosolic Ca²⁺ concentration, and the down-regulated ones to ion transport and cell homeostasis (Fig. 10D). This activity was associated with a higher edema accumulation and virus lethality. Among these genes, that of Cystic Fibrosis Transmembrane Regulator (CFTR) was exclusively down-regulated due to the presence of the E protein PBM from SARS-CoV and SARS-CoV-2. CFTR is a key factor in the edema resolution, which is impaired in lung infected mice with PBM-SARS-2. SARS-CoV-2 infection also led to lung edema, in comparison with a virus without E PBM motif (Fig. 16).

SARS-CoV and SARS-CoV-2 infection reduced CFTR mRNA levels in both Calu-3 2b4 cells and in the lung of infected mice, in relation with the levels in cell culture or in mice infected with the respective mutant without E PBM motif (Fig. 11).

*CFTR modulators regulated virus production in infected cells.*

CFTR is a key factor for lung edema resolution. Two compounds, a corrector (VX809) and a potentiator (VX770) modulate CFTR localization and function and have been used in combination to treat cystic fibrosis. We administered non-toxic concentrations of two CFTR modulators (VX809 or VX770) (Fig. 17) to Calu-3-2B4 cells and mice infected with SARS-CoV-2. Those treated with the combination of CFTR modulators showed a reduction of 10 and 7-fold the viral titers *in vitro* and *in vivo,* respectively (Fig. 12). In addition, it was shown that the administration of CFTR modulators VX809, or VX770, alone or a combination of both drugs to SARS-CoV-2 infected K18-hACE2 mice, led to protection levels of 20, 40 or 80%, respectively (Fig.13). These results demonstrate the therapeutic effect of both VX809 and VX770 separately (20% and 40% survival) and the synergy of a combination of both compounds (80% survival).

### Effect of CFTR modulators on inflammatory cytokines induced by SARS-CoV-2 in lungs of infected mice

SARS-CoV-2 E protein modulates NLRP3 inflammasome, the effect of CFTR modulators on lung NLRP3 and NLRP3 dependent cytokines (TNF-α, IL-6, IL-1β) and chemokines (CXCL10 and CXCL2) mRNA levels were analyzed in SARS-CoV-2 infected mice by qPCR (Fig. 14). SARS-CoV-2 infection induced higher levels of the inflammasome NLRP3, cytokines and chemokines at 3 dpi. In contrast, lungs from mice infected and treated with the combination of CFTR modulators showed lower levels of NLRP3 and NLRP3 dependent cytokines and chemokines (Fig. 14). Therefore, CFTR modulators have an anti-inflammatory effect during SARS-CoV-2 infection.

## Claims

1. A CFTR modulator compound selected from the group consisting of 3-[6-[[1-(2,2-difluoro-1,3-benzodioxol-5-yl)cyclopropanecarbonyl]amino]-3-methylpyridin-2-yl]benzoic acid and/or N-(2,4-ditert-butyl-5-hydroxyphenyl)-4-oxo-1H-quinoline-3-carboxamide and pharmaceutically acceptable salts or deuterated derivatives thereof for use in the treatment of an infection produced by a Betacoronavirus in a subject.

2. The CFTR modulator compound according to claim 1, being a combination of 3-[6-[[1-(2,2-difluoro-1,3-benzodioxol-5-yl)cyclopropanecarbonyl]amino]-3-methylpyridin-2-yl]benzoic acid and N-(2,4-ditert-butyl-5-hydroxyphenyl)-4-oxo-1H-quinoline-3-carboxamide.

3. The CFTR modulator compound according to claim 1 or 2, wherein the concentration of 3-[6-[[1-(2,2-difluoro-1,3-benzodioxol-5-yl)cyclopropanecarbonyl]amino]-3-methylpyridin-2-yl]benzoic acid is between 1000 mg/kg and 1 mg/kg, preferably, between 500 mg/kg and 2 mg/kg, more preferably between 100 mg/kg and 3 mg/kg, even more preferably between 50 mg/kg and 4 mg/kg and even more preferably between 25 mg/kg and 5 mg/kg.

4. The CFTR modulator compound according to claim 1 or 2, wherein the concentration of N-(2,4-ditert-butyl-5-hydroxyphenyl)-4-oxo-1H-quinoline-3-carboxamide is between 1000 mg/kg and 1 mg/kg, preferably between 500 mg/kg and 2 mg/kg, more preferably between 100 mg/kg and 3 mg/kg, even more preferably between 50 mg/kg and 4 mg/kg and even more preferably between 25 mg/kg and 5 mg/kg.

5. The CFTR modulator compound according to any of the preceding claims, wherein the concentration of 3-[6-[[1-(2,2-difluoro- 1,3-benzodioxol- 5-yl) cyclopropanecarbonyl]amino]-3-methylpyridin-2-yl]benzoic acid is between 25 mg/kg and 5 mg/kg and the concentration of N-(2,4-ditert-butyl-5-hydroxyphenyl)-4-oxo-1H-quinoline-3-carboxamide is between 25 mg/kg and 5 mg/kg.

6. The CFTR modulator compound according to any of the preceding claims, wherein the administration happens before the subject shows the symptoms of a Betacoronavirus infection.

7. The CFTR modulator compound according to any of the preceding claims, wherein the infection produced by a Betacoronavirus in a subject produces a respiratory pathology or lung pathology.

8. The CFTR modulator compound according to any of the preceding claims, wherein the Betacoronavirus has a PBM domain formed by the last four amino acids of the carboxy terminus of the E protein selected from the group consisting of:
- class I PBM X-S/T-X-Φ_{-COOH} sequence,
- class II PBM X- Φ-X-Φ_{-COOH} sequence or
- class III PBM X-D/E-X-Φ_{-COOH} sequence,
wherein X is any amino acid and Φ is a hydrophobic amino acid, preferably V, ! or L.

9. The CFTR modulator compound according to the preceding claim, wherein the PBM domain has the sequence -DLLV-_{COOH}.

10. The CFTR modulator compound according to any one of claims 1 to 8, wherein the Betacoronavirus is selected from the group consisting of SARS-CoV, MERS-CoV and SARS-CoV-2.

11. The CFTR modulator compound according to claims any one of 1 to 10, wherein the subject is a vertebrate, preferably, a mammal selected from the group consisting of human, horse, pig, rabbit, dog, sheep, goat, non-human primate, cow, cat, guinea pig or rodent.

12. The CFTR modulator compound according to any of the preceding claims, for administration to a subject topically, intranasally, orally, subcutaneously, intraperitoneally or intramuscularly.

13. A pharmaceutical composition comprising the one or more of the CFTR modulators, pharmaceutically acceptable salt thereof, or deuterated derivative thereof, as defined in claims 1 to 12, and:
one or more additional active compound, and/or
one or more pharmaceutically acceptable carrier, adjuvant, or vehicle for use in the treatment of an infection produced by a Betacoronavirus in a subject.

14. The pharmaceutical composition according to the preceding claim, wherein the additional active compound is selected from a mucolytic agent, bronchodilator, an anti-biotic, an anti-viral agent, an anti-inflammatory agent, or a nutritional agent.

15. The pharmaceutical composition according to the preceding claim, wherein the anti-viral agent is selected from one or more of the group consisting of an adenosine analog that inhibits viral replication, a protease inhibitor, a ribonucleoside, chloroquine, hydroxycholorquine, interferon beta, interferon alpha and combinations thereof.
